# EUROPEAN PATENT SPECIFICATION

(11) **EP 3 608 393 B1**
(45) Date of publication and mention of the grant of the patent: **28.12.2022**
(21) Application number: 19190162.8
(22) Date of filing: 06.08.2019
(51) Int. Cl.: C12G 1/022, C12G 1/028, C12M 1/12, C12M 1/02, C12C 11/00, F28D 1/06, F28D 21/00

(54) **TANK WITH SPIRAL HEAT-EXCHANGE JACKET**
TANK MIT SPIRALWÄRMEAUSTAUSCHMANTEL
RÉSERVOIR AVEC GAINE D'ÉCHANGE THERMIQUE EN SPIRALE

(30) Priority: 07.08.2018 IT 201800007924
(43) Date of publication of application: 12.02.2020
(73) Proprietor: Biasior, Patrizia, 38123 Trento (IT)
(72) Inventor: Biasior, Patrizia, 38123 Trento (IT)
(74) Representative: Zelioli, Giovanni

(56) References cited:
- EP-A2- 1 114 672
- WO-A1-2017/191192
- CN-U- 203 360 420
- DE-C- 969 963
- US-A1- 2017 335 256

## Description

### Technical field

The present invention refers to a tank having a substantially cylindrical body.

In general, the present invention can be applied in the field of tanks for beverage liquids such as wine or beer, or non-food liquids such as tanks for the gas/oil sector.

### Background art

There exist tanks for containing liquids that are called "fermentation tanks". Such fermentation tanks can be used for containing, in particular, food liquids, such as wine, beer, etcetera.

In the known fermentation tanks, a temperature control is provided during fermentation, by means of refrigerant jackets positioned outside the tank, on the outer wall, so as to refrigerate also the inner wall and thus the liquid content of the tank.

The refrigerant jackets can be installed vertically or horizontally, in one or more sections. Typically, the refrigerant jackets are installed by welding on the lateral wall of the refrigerant tank.

Document US2005077029 (A1) refers to a fermentation tank, in particular for beer, wherein, in order to improve the temperature control, a heat exchanger is used positioned internally at the center of the tank, arranged vertically.

Document CN103822504 (A) refers to a fermentation tank comprising a refrigerant jacket, which comprises a jacket body whose outer portion is provided with a covering portion. The covering portion is arranged in a spiral-like manner on the outer portion of the jacket body, so as to form a spiral-like tubular passage within which the refrigerant fluid circulates. Document CN103822504 (A) therefore proposes to increase the thermal exchange area by means of the spiral-like passage, to improve the refrigerating efficiency.

Document US2017335256 (A1) relates to a beverage making apparatus including a fermentation tank assembly having an opening, a fermentation tank cover covering the opening, and a heat exchange element arranged in contact with the fermentation tank.

Document DE969963 (C) relates to a beer fermentation vat or storage tank with concrete cladding and cooling pipes laid in the cladding, which at the same time serve to stiffen the walls and to improve heat or cold transmission.

Document CN203360420 (U) relates to jacketed tank comprising a barrel, a jacket, a heat preservation layer, and a housing, wherein the heat preservation layer is arranged between the jacket and the housing; the spiral jacket is arranged between the barrel and the heat preservation layer and connected with the heat preservation layer; the jacket is spiral and is welded on the heat preservation layer, so that the cooling effect is improved.

Nevertheless, the solutions of known tanks present some disadvantages.

In the first place, the tanks comprise a lateral wall realized through metal sheet welding. Therefore, they are sensitive to production defects, as the weldings can be a source of encrustations due to the contained liquid or can be the source of breakages of the shell plating of the tank, because of a localized decrease of structural resistance.

In the second place, the tanks with refrigerant jackets involve thermal transfer which may be not fully effective. The thermal yield is therefore not predictable and imposes a lower temperature in use for the refrigerants, generating higher operating costs for the whole life of the equipment and quite a biological damage to the fermentation yeasts.

In the third place, in particular in presence of refrigerant jackets, the production of tanks results to be delicate and expensive, and possible defects in the assembly of the lateral wall and in the application of the refrigerant jackets, have repercussions on the global performances of the system.

### Summary of the invention

An objective of the present invention is to solve inconveniences of the background art.

A general objective of the present invention is to present a tank having such a configuration as to simplify the production thereof and at the same time improve the yield thereof when applied to a fermenting content or a content that needs a temperature control.

A particular objective of the present invention is to present a tank comprising a lateral wall realized by welding of metal sheet, which is structurally resistant and of better quality.

A further particular objective of the present invention is to present a tank comprising refrigerant jackets, which is more efficient as regards thermal transfer, in particular concerning a food liquid content fermenting in the tank.

A further particular objective of the present invention is to present a tank comprising refrigerant jackets whose production is more efficient and rational, to decrease incidence of possible assembly defects.

These and other objectives are achieved by means of the features of the annexed claims that are an integral part of the present description.

The present invention provides a tank, in particular a fermentation tank, having a substantially cylindrical body comprising a lateral wall.

The lateral wall is constituted by metal sheet wound according to a three-dimensional spiral, wherein the metal sheet comprises a main development direction and two lateral edges aligned with respect to the main development direction; the two lateral edges comprise first segments welded with second segments of the two lateral edges respectively, said first segments and said second segments thus being adjacent in the three-dimensional spiral for closing the lateral wall.

The tank further comprises at least one heat-exchange jacket, associated with said lateral wall for circulation of a fluid inside said heat-exchange jacket, so as to exchange heat through the lateral wall with a content of said tank.

Preferably, the at least one heat-exchange jacket is arranged on the metal sheet and is aligned with respect to the main development direction, thus being wound according to the three-dimensional spiral. In particular, the at least one heat-exchange jacket comprises a plurality of turns on the lateral wall, along the three-dimensional spiral, preferably comprising at least one complete turn. In particular, the heat-exchange jacket is applied to the metal sheet.

Advantageously, the tank of the present invention is realized in a more efficient and rational manner.

Advantageously, the lateral wall of the tank according to the present invention results to be structurally resistant and of optimal quality.

Preferably, the tank further comprises a supply line hydraulically connected to the at least one heat-exchange jacket for inflow of the fluid, and further comprises a return line hydraulically connected to the at least one heat-exchange jacket for outflow of the fluid, wherein the heat-exchange jacket develops from an upper level of the tank to a lower level of the tank, and wherein the supply line is connected to the at least one heat-exchange jacket at the upper level, and wherein the return line is connected to the at least one heat-exchange jacket at the lower level.

Advantageously, the tank of the present invention results to be more efficient as regards thermal transfer.

In general, advantageously, the tank of the present invention allows to realize a heat-exchange jacket with better thermodynamic performances, obtaining greater efficiency of thermal transfer, up to 60% more with respect to traditional tanks, also thanks to the minor thickness of the lateral wall with equal structural resistance.

Advantageously, the tank of the present invention allows lower operational costs, during the whole service life of the tank.

Advantageously, the tank of the present invention allows an easier cleaning of the inner walls, thereby allowing even automatic sanitization and lower maintenance costs.

Advantageously, the tank of the present invention exhibits a greater rigidity of the shell plating that constitutes the lateral wall, both when the tank is stressed under pressure and when it is vacuum stressed, or when the tank undergoes seismic movements.

Advantageously, the tank according to the present invention does not present any horizontal welding, which could be the source of encrustations.

Advantageously, the tank according to the present invention does not present any vertical welding, which could be the source of breakages in the shell plating that constitutes the lateral wall.

Advantageously, the tank according to the present invention does not require intersections in the weldings, thus avoiding potential critical breaking points.

Advantageously, the tank according to the present invention provides for an interstices-free construction.

Preferably, it is provided a metal sheet having a distance between the two lateral edges that is greater than 1000 mm, preferably substantially equal to or greater than 1250 mm but not limited to such size. According to a correlated aspect of the present invention, it is provided a metal sheet also having a thickness greater than 3 mm, preferably equal to or greater than 4mm.

Advantageously, the tank according to the present invention can be manufactured by means of an automated continuous procedure, thus reducing the incidence of possible assembly defects. In particular, the welding process can occur in an automatic manner and in continuous, without requiring a preliminary spot welding of the components and without any interruption of the productive process.

Advantageously, the tank according to the present invention can provide for an automatic cold rolling of the weldings of the shell plating.

Further features and advantageous will become apparent from the detailed description made hereinafter of preferred but not limiting embodiments of the present invention, and from the dependent claims that delineate preferred and particularly advantageous embodiments of the invention.

### Brief description of drawings

The invention is illustrated with reference to the following figures, provided as a way of non-limiting examples, wherein:
- Figures 1A, 1B and 1C illustrate views of a tank according to a first embodiment of the present invention.
- Figure 2 schematically illustrates a first embodiment of a fluid circuit in a tank according to the first embodiment of the present invention.
- Figures 3A, 3B and 3C illustrate views of a tank according to a second embodiment of the present invention.
- Figure 4 schematically illustrates a second embodiment of a circuit of fluid in a tank according to a further embodiment of the present invention.

In the different figures, similar elements will be identified with similar reference numbers.

### Detailed description

The present description will refer to a tank, as an illustrative and not limiting embodiment of the present invention.

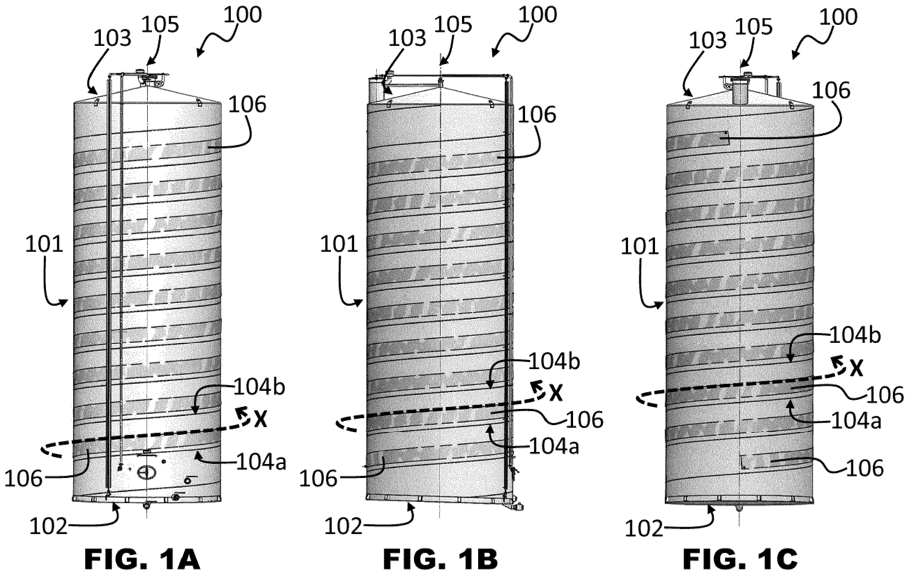

Figures 1A, 1B and 1C respectively illustrate a front view, a lateral view and a back view of a tank 100 according to the present invention.

The tank 100 has a substantially cylindrical body, in particular upright in a working position. The tank 100 comprises a lateral wall 101, which laterally envelops it and defines the enclosed inner volume thereof. The tank 100 also comprises a bottom plate 102 and a top plate 103, which contribute to the closure of the structure. The tank 100 further comprises accessory elements such as ducts, portholes, vents, etcetera within knowledge of the skilled in the art and that are not herein described for sake of brevity.

In one embodiment, the lateral part 101 is constituted by metal sheet, wound according to a three-dimensional spiral. In particular, the metal sheet comprises steel, stainless or not, in particular adapted to the contact with food and non-food liquids.

The metal sheet comprises a main development direction X. The metal sheet has two lateral edges 104a and 104b, aligned with respect to the main development direction X.

In particular, the two lateral edges 104a and 104b comprise first segments welded to second segments of said two lateral edges 104a and 104b respectively; in fact, the first segments and the second segments of the two lateral edges 104a and 104b result to be respectively adjacent in the three-dimensional spiral of the lateral wall 101, so as to close it, being wound clockwise or counter-clockwise.

The metal sheet provides for a distance between the two lateral edges 104a and 104b that is greater than 1000 mm. Preferably, the distance between the two lateral edges 104a and 104b is, substantially, but not in a limiting way, equal to or greater than 1250 mm.

Tanks of the background art are, in fact, produced using metal sheet having a height of max. 1 meter. By applying a metal sheet greater than 1 m, i.e. greater than 1000 mm, the meters associated with the lateral edges 104a and 104b to be linearly welded are reduced, for a same final size of the tank 100. In this manner, it is possible to increase the quality and at the same time reducing production costs.

In particular, the metal sheet is a rolled coil, not being the object of a subsequent edging along the two lateral edges 104a and 104b, before being assembled in the tank. Such sheet material is constituted by a metal sheet coil, and originally wound along the main development direction X.

Further, in particular, the metal sheet has a thickness greater than 3 mm, preferably, equal to or greater than 4mm. Such a thickness greater than 3 mm is obtained by means of rolling a sheet material actually having a starting thickness greater than 3 mm.

In general, roll-bending and rolling are processes that provide for a plastic deformation of metal sheet plates or coils.

In the present invention, preferably, a machinery called "calender" is used for this manufacture. Such machinery is provided with a plurality of rolls with parallel axes, preferably with vertical axes, arranged in such a way that the metal sheet coil, passing through between them, follows a circular trajectory that is in accordance with the diameter of the tank to be obtained.

Tanks of the prior art are produced using metal sheet coils having a thickness minor than or equal to 3 mm. By applying a metal sheet coil of a greater thickness, it is possible to increase the resistance of the tank 100 to pressure and vacuum stresses, also according to technical regulations of the field. In this way, it is possible to increase the performances and the maximum dimensions that can be achieved for the tank, making it suitable for greater and heavier applications.

As it is evident for the skilled in the art, the dimensions of the sheet that are indicated in millimeters are to be considered including an appropriate dimensional production tolerance of the tape. Such dimensional tolerance can cover up to 5% more or less as regards the distance between the lateral edges, and up to 10% more or less as regards the thickness of the metal sheet.

During assembly of the tank 100, the lateral edges 104a and 104b are aligned along the direction X, within a production tolerance accounting for possible dimensional defects of the raw material. Eventual misalignments of the lateral edges 104a and 104b can be appropriately corrected during a welding phase of the respective segments, as described above.

The result is that the two lateral edges 104a and 104b are coupled and respectively welded, being wound according to the three-dimensional spiral. Preferably, but not in a limiting way, the lateral edges 104a and 104b are welded in an automated way, by means of TIG or Plasma or Laser welding. The lateral edges 104a and 104b are respectively welded with a Class 1 certified welding according to the EN ISO 13445 standard, or an equivalent one.

The result is also that the three-dimensional spiral that constitutes the lateral wall 101 has a helix pitch equal to or greater than the distance between the two lateral edges 104a and 104b. In a preferred embodiment, the three-dimensional spiral that constitutes the lateral wall 101 has a helix angle, with respect to an axis perpendicular to the vertical 105 of the tank 100 that proportionally depends on the diameter and on the height of the metal sheet coil used.

In a non-limiting example, such a helix angle is comprised between 2° and 10°, preferably comprised between 2° and 4°, more preferably equal to 3°.

The continuous three-dimensional spiral construction of the tank 100 does not require a previous spot welding, and couples the edges 104a and 104b in an automatic way by means of an adjustable guide system. The welding of the edges 104a and 104b is made with controlled parameters, and the whole process can be periodically certified complying with the EN ISO regulations.

Further, the continuous three-dimensional spiral construction of the lateral wall 101 eliminates all the intersections of the shell plating junctions, assuring a quality standard of absolute importance and better than the one that can be obtained by means of the background art technologies.

The continuous three-dimensional spiral welding results also to be a reinforcing structural element in the shell plating of the lateral wall 101 of the tank 100: this is of particular importance for application of external pressure to the tank 100, or in case of internal vacuum in the tank 100, which can happen in an unloading phase of the liquid product contained.

In one embodiment, the tank 100 comprises at least a heat-exchange jacket 106, associated with the lateral wall 101. In the example of the tank 100, a single heat-exchange jacket 106 is provided, realized in continuous.

The heat-exchange jacket 106 is used for the circulation of a fluid inside it, so as to exchange heat through the lateral wall 101 with a content of the tank 100.

The heat-exchange jacket 106 is arranged on the metal sheet and aligned with respect to the main development direction X, thus also being wound according to the same three-dimensional spiral that constitutes the lateral wall 101.

In other embodiments, not shown, a tank could comprise two or more mutually parallel lateral jackets, or a plurality of sections of lateral jackets at mutual intervals along the main development direction X.

The heat-exchange jacket 106 comprises a plurality of turns on the lateral wall 101, along the three-dimensional spiral, preferably comprising at least one complete turn or, as in the represented case, more than one complete turn.

The heat-exchange jacket 106 is applied to the metal sheet by means of a continuous or discontinuous process, in particular an automated process. In particular, two lateral edges 104a and 104b of the metal sheet are welded for constituting the lateral wall 101, when the metal sheet already comprises the heat-exchange jacket 106 on an outer side, previously applied to it.

The heat-exchange jacket 106, in particular but not in a limiting way, is exploited for the production and fermentation of wine.

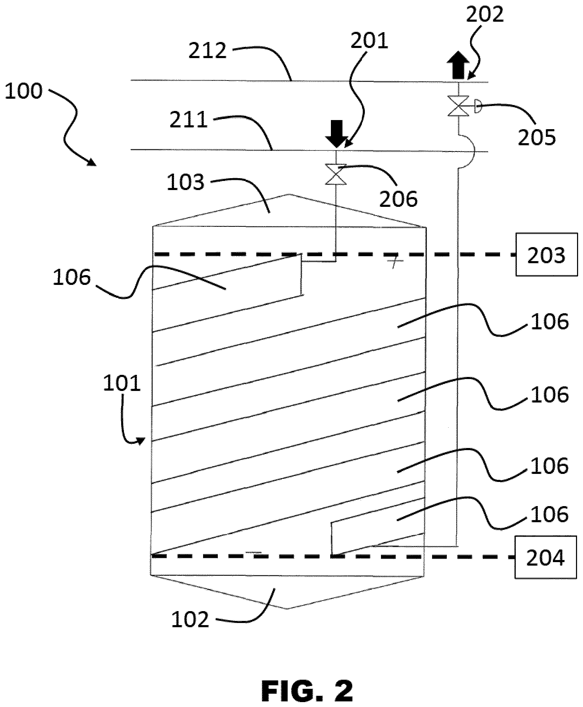

Figure 2 schematically illustrates the fluid circuit in the heat-exchange jacket 106 of the tank 100.

The tank 100 can comprise a supply line 201, hydraulically connected to the heat-exchange jacket 106, for inflow of a heat-exchange fluid. The tank 100 further comprises a return line 202, hydraulically connected to the heat-exchange jacket 106 for outflow of the fluid.

The heat-exchange jacket 106, as can be seen, develops from an upper level 203 of the tank 100, to a lower level 204 of the tank 100. The supply line 201 is connected to the heat-exchange jacket 106 at the upper level 203, while the return line 202 is connected to the heat-exchange jacket 106 at the lower level 204. As it is evident, the upper level 203 is located, on the lateral wall 101, at a height that is greater with respect to a height at which the lower level 204 is located, when the tank 100 is upright in a working position.

This particular configuration of the supply line 201 and of the return line 202 associated with the heat exchange jacket 106, allows to create a fluid flow, be it of a refrigerant or of a heating fluid, maximizing a temperature difference between the fluid circulating in the heat-exchange jacket 106 and the product to be conditioned and contained in the tank 100; further, for any level of product contained in the tank 100, it becomes possible to control the temperature efficiently.

Given that, for the content of the tank 100, a hotter food fluid tends to rise with respect to a colder food fluid, different temperature stratifications are verified, with higher temperatures in the upper level 203 and lower temperatures in the lower level 204.

Such a problem is very significant in the case of fermentation processes, for example of wines or beers, where temperature has a significant effect on the behaviour of microorganisms, i.e. yeasts; in these cases, different temperatures yield to different final products, requiring transfers of liquid from the lower part to the upper part of the tank, typically made by means of pumps that, apart from creating cavitation damage, result in increases of operational costs and risks of loss of food product.

Providing a supply line 201 (for the inlet of the fluid) connected to the heat-exchange jacket 106 at the upper level 203, and a return line 202 (for the outlet of the fluid) connected to the heat-exchange jacket 106 at the lower level 204, it is possible to obtain a refrigerant fluid that at the upper level 203 is at a lower temperature and that at the lower level 204 is at a higher temperature, thus allowing a more efficient refrigeration of the content of the tank, preferably of a fermentation liquid.

Such an effect significantly improves the thermal transfer between the thermal exchange jacket 106 and the content of the tank 100, generating also energy savings because the increased temperature difference between the refrigerant fluid and the content in fermentation, also increases heat flux. In this situation, a minor flow rate of refrigerant fluid or a less cold temperature thereof is enough to modify and stably maintain the temperature of the contained product.

In general, the supply line 201 is connected to a main supply line 211, and the return line 202 is connected to a main return line 212. The main supply line 211 and the main return line 212 allow to operatively connect the circuits of more than one tank, in parallel.

Preferably, the tank 100 further comprises a valve 205 with automatic shut-off on the return line 202, and comprises a valve 206 without automatic shut-off on the supply line 201.

The main supply line 211 and the valve 206 are configured for allowing outflow of gas in continuous. In this way, inlet of refrigerant fluid is allowed without passing through a valve with automatic shut-off, thus allowing the upper level 203 to function as a continuous discharge for eventual gases released by the fluid, which thus does not accumulate in the heat-exchange jacket 106.

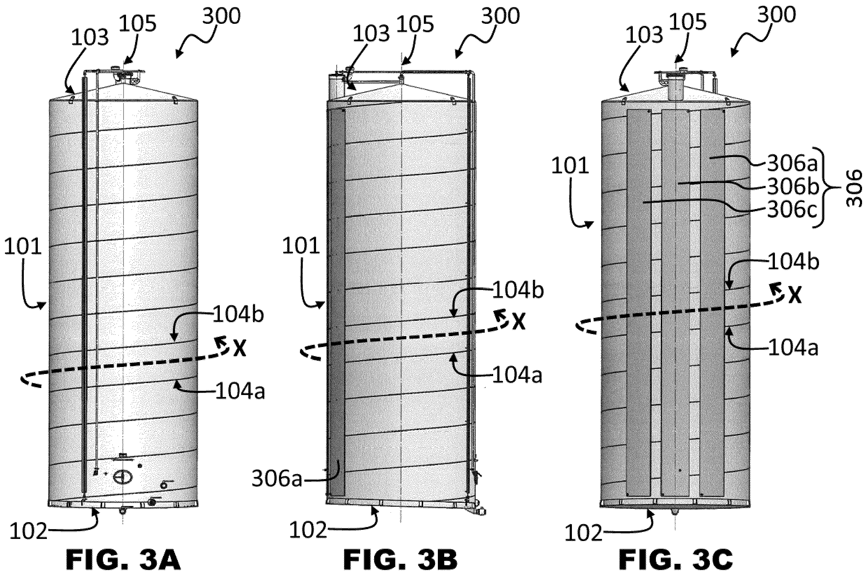

Figures 3A, 3B and 3C illustrate a front view, a lateral view and a back view, respectively, of a further tank 300 according the present invention.

The tank 300 has a construction of the body that is substantially analogous to that of the tank 200, already described above, and integrates analogous technical features that are not all repeated below, but that are to be intended as anyway included.

In particular, the tank 300 has a substantially cylindrical body that comprises a lateral part 101, which envelops it laterally and that defines the inner volume enclosed thereof. The lateral part 101 is constituted by metal sheet, wound according to a three-dimensional spiral; the metal sheet comprises a main development direction X. The metal sheet has two lateral edges 104a and 104b, aligned with respect to the main development direction X.

In particular, the two lateral edges 104a and 104b comprise first segments respectively welded with second segments of said two lateral edges 104a and 104b; in fact, the first segments and the second segments of the two lateral edges 104a and 104b result to be respectively adjacent in the three-dimensional spiral of the lateral wall 101, so as to close it.

The metal sheet provides for a distance between the two lateral edges 104a and 104b that is greater than 1000 mm, preferably substantially equal to 1250 mm.

It results that the two lateral edges 104a and 104b are coupled and respectively welded, being wound according to the three-dimensional spiral.

Further, in particular, the metal sheet has a thickness greater than 3 mm, preferably equal to or greater than 4 mm. Such a thickness greater than 3 mm is possible by means of roll-bending raw material, called tape or coil, actually having a starting thickness greater than 3 mm.

In another embodiment, the tank 300 comprises three heat-exchange jackets 306a, 306b and 306c, indicated collectively with the reference 306, which are associated with the lateral wall 101. In particular, the heat-exchange jackets 306a, 306b and 306c are applied to the tank 300 after the realization of the lateral wall 101, or in a process not in continuous, for example, by means of welding.

The heat-exchange jackets 306a, 306b and 306c are used for the circulation of a fluid inside it, so as to exchange heat through the lateral wall 101 with a content of the tank 300.

In particular, the heat-exchange jackets 306a, 306b and 306c are arranged vertically when the tank 300 is upright in a working position.

In other embodiments, not shown, a tank could comprise a different number of vertical heat-exchange jackets 306, one or more than one. In still other embodiments, not shown, a tank could comprise a different number of sections of heat-exchange jackets 306, one or more than one, aligned vertically and at intervals along the vertical development of the tank 300.

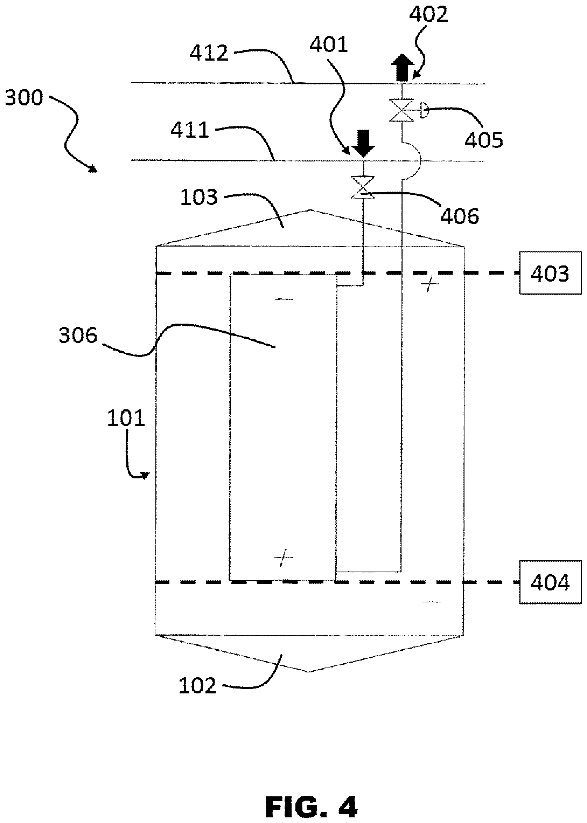

Figure 4 schematically illustrates the circuit of fluid in a vertical heat-exchange jacket 306 of the tank 300.

It is to be intended that the configuration of the circuit of fluid associated with the heat-exchange jacket 306 is applicable to a generic tank comprising a lateral wall 101, also not realized by means of metal sheet wound according to a three-dimensional spiral, as described with reference to the tanks 100 and 300.

The tank 300 comprises a supply line 401, hydraulically connected to the heat-exchange jacket 306, for inflow of a heat-exchange fluid. The tank 300 further comprises a return line 402, hydraulically connected to the heat-exchange jacket 306 for outflow of the fluid.

The heat-exchange jacket 306, as it can be seen, develops from an upper level 403 of the tank 300, to a lower level 404 of the tank 300. The supply line 401 is connected to the heat-exchange jacket 306 at the upper level 403, while the return line 402 is connected to the heat-exchange jacket 306 at the lower level 404. As it is evident, the upper level 403 is located on the lateral wall 101, at a height that is greater with respect to a height where the lower level 404 is located, when the tank 300 is upright and in a working position.

This particular configuration of the supply line 401 and of the return line 402 associated with the heat-exchange jacket 306, allows to create a flow of fluid, be it of a refrigerant or of a heating fluid, maximizing a temperature difference between the circulating fluid in the heat-exchange jacket 306 and the product to be conditioned and contained in the tank 300; further, for any level of product contained in the tank 300 it becomes possible to control the temperature efficiently.

In general, the supply line 401 is connected to a main supply line 411, and the return line 402 is connected to a main return line 412. The main supply line 411 and the main return line 412 allow to operatively connect the circuits of more than one tank, in parallel.

Preferably, the tank 300 further comprises a valve 405 with automatic shut-off on the return line 402, and comprises a valve 406 without automatic shut-off on the supply line 401. The main supply line 411 and the valve 406 are configured to allow the outflow of gas in continuous.

### Industrial applicability

Advantageously, the lateral wall of the tank according to the present invention results to be structurally resistant and of optimal quality.

Advantageously, the tank of the present invention is realized in a more efficient and rational manner, reducing the incidence of possible assembly defects of the heat-exchange jacket and reducing production costs and expenses.

Advantageously, the tank of the present invention results to be more efficient as regards thermal transfer, resulting to be particularly suitable for holding a food liquid content in fermentation, such as wine or beer.

During the thermal exchange with the content of the tank, vertical fluiddynamic currents are created capable of mixing it, thus avoiding undesired settlings of deposits or microorganisms and increasing thermal transfer.

Considering the description herein made, the skilled in the art will be able to devise further modifications and variants, so as to satisfy contingent and specific needs.

It is evident that, where there are no evident technical incompatibilities for the skilled in the art, the configurations of specific elements described with reference to some embodiments may be used in other embodiments herein described.

The invention can be applied to tanks which are different from the fermentation tanks, for food liquids or other non-food liquids. The heat-exchange jackets can be exploited for production, refrigeration and maturation of beer, of other alcoholic beverages and even for the production of milk or dairy products.

In that sense, the present invention can be applied also to storage tanks, or other types of tank.

The embodiments described here are therefore to be intended as illustrative and not limiting examples of the invention.

## Claims

1. Tank (100, 300), in particular fermentation tank, having a substantially cylindrical body comprising:
a lateral wall (101) constituted by metal sheet wound according to a three-dimensional spiral,
wherein said metal sheet comprises a main development direction (X) and two lateral edges (104a, 104b) aligned with respect to said main development direction (X), said two lateral edges (104a, 104b) comprising first segments welded with second segments of said two lateral edges (104a, 104b) respectively, said first segments and said second segments being adjacent in said three-dimensional spiral for closing said lateral wall (101),
said tank (100, 300) further comprising at least one heat-exchange jacket (106, 306) associated with said lateral wall (101), for circulation of a fluid inside said heat-exchange jacket (106, 306), so as to exchange heat through said lateral wall (101) with a content of said tank (100, 300).

2. Tank according to claim 1, wherein said at least one heat-exchange jacket (106, 306) is arranged on said metal sheet and is aligned with respect to said main development direction (X), thus being wound according to said three-dimensional spiral.

3. Tank according to claim 2, wherein said at least one heat-exchange jacket (106, 306) comprises a plurality of turns on said lateral wall (101), along said three-dimensional spiral, preferably comprising at least one complete turn.

4. Tank according to any one of claims 1 to 3, wherein said heat-exchange jacket (106, 306) is applied to said metal sheet, in particular being welded.

5. Tank according to claim 4, wherein said two lateral edges (104a, 104b) of said metal sheet are subsequently welded for constituting said lateral wall (101), said sheet already comprising said heat-exchange jacket (106, 306) on an outer side.

6. Tank according to any one of claims 1 to 5, wherein said two lateral edges (104a, 104b) are coupled being wound according to said three-dimensional spiral and welded, preferably in continuous, preferably in an automated way and preferably by TIG or Plasma or Laser welding.

7. Tank according to any one of claims 1 to 6, wherein said three-dimensional spiral has a helix pitch equal to or greater than a distance between said two lateral edges (104a, 104b).

8. Tank according to any one of claims 1 to 7, further comprising a supply line (201, 401) hydraulically connected to said at least one heat-exchange jacket (106, 306) for inflow of said fluid, and further comprising a return line (202, 402) hydraulically connected to said at least one heat-exchange jacket (106, 306) for outflow of said fluid, wherein said heat-exchange jacket (106, 306) develops from an upper level (203, 403) of said tank (100, 300) to a lower level (204, 404) of said tank (100, 300), and wherein said supply line (201, 401) is connected to said at least one heat-exchange jacket (106, 306) at said upper level (203, 403), and wherein said return line (202, 402) is connected to said at least one heat-exchange jacket (106, 406) at said lower level (204, 404).

9. Tank according to claim 8, wherein said upper level (203, 403) is located on said lateral wall (101) at a height that is greater with respect to a height at which said lower level (204, 404) is located, when said tank (100, 300) is upright in a working position.

10. Tank according to claim 8 or 9, further comprising a first valve (205, 405) with automatic shut-off on said return line (202, 402).

11. Tank according to claim 10, further comprising a main supply line (211, 411) connected to said supply line (201, 401), and further comprising a second valve (206, 406) on said supply line (201, 401), said main supply line (211, 411) and said second valve (206, 406) being configured for allowing outflow of gas in continuous through said second valve (206, 406) and said main supply line (211, 411).

12. Tank according to any one of claims 8 to 11, wherein said fluid is a refrigerant fluid which, at said upper level (203, 403), is at a lower temperature and which, at said lower level (204, 404), is at a higher temperature, after having refrigerated a content of said tank (100, 300), preferably a fermenting liquid.

13. Tank according to any one of claims 1 to 12, wherein said metal sheet is a metal sheet coil wound along said main development direction (X).

## Patentansprüche

1. Tank (100, 300), insbesondere Fermentationstank, der einen im Wesentlichen zylindrischen Körper aufweist, wobei er Folgendes umfasst:
eine seitliche Wand (101), die durch Metallblech gebildet ist, das entsprechend einer dreidimensionalen Spirale gewickelt ist,
wobei das Metallblech eine Hauptentwicklungsrichtung (X) und zwei seitliche Kanten (104a, 104b) die in Bezug auf die Hauptentwicklungsrichtung (X) ausgerichtet sind, umfasst, wobei die zwei seitlichen Kanten (104a, 104b) erste Segmente umfassen, die jeweils mit zweiten Segmenten der zwei seitlichen Kanten (104a, 104b) verschweißt sind, wobei die ersten Segmente und die zweiten Segmente in der dreidimensionalen Spirale benachbart sind, um die seitliche Wand (101) zu schließen,
wobei der Tank (100, 300) ferner mindestens einen Wärmeaustauschmantel (106, 306) umfasst, der mit der seitlichen Wand (101) verknüpft ist, zum Umlauf eines Fluids innerhalb des Wärmeaustauschmantels (106, 306), um so durch die seitliche Wand (101) Wärme mit einem Inhalt des Tanks (100, 300) auszutauschen.

2. Tank nach Anspruch 1, wobei der mindestens eine Wärmeaustauschmantel (106, 306) auf dem Metallblech angeordnet ist und in Bezug auf die Hauptentwicklungsrichtung (X) ausgerichtet ist, wobei er folglich entsprechend der dreidimensionalen Spirale gewickelt ist.

3. Tank nach Anspruch 2, wobei der mindestens eine Wärmeaustauschmantel (106, 306) eine Vielzahl von Windungen auf der seitlichen Wand (101), entlang der dreidimensionalen Spirale, umfasst, wobei er vorzugsweise mindestens eine vollständige Windung umfasst.

4. Tank nach einem der Ansprüche 1 bis 3, wobei der Wärmeaustauschmantel (106, 306) auf das Metallblech aufgebracht ist, wobei er vorzugsweise verschweißt ist.

5. Tank nach Anspruch 4, wobei die zwei seitlichen Kanten (104a, 104b) des Metallblechs nacheinander verschweißt sind, um die seitliche Wand (101) zu bilden, wobei das Blech auf einer äußeren Seite bereits den Wärmeaustauschmantel (106, 306) umfasst.

6. Tank nach einem der Ansprüche 1 bis 5, wobei die zwei seitlichen Kanten (104a, 104b) gekoppelt sind, wobei sie entsprechend der dreidimensionalen Spirale gewickelt und, vorzugsweise kontinuierlich, vorzugsweise auf eine automatisierte Weise und vorzugsweise durch WIG- oder Plasma- oder Laserschweißen, verschweißt sind.

7. Tank nach einem der Ansprüche 1 bis 6, wobei die dreidimensionale Spirale eine Wendelganghöhe aufweist, die gleich einem Abstand zwischen den zwei seitlichen Kanten (104a, 104b) oder größer als derselbe ist.

8. Tank nach einem der Ansprüche 1 bis 7, der ferner eine Zuführungsleitung (201, 401) umfasst, die zum Einströmen des Fluids hydraulisch mit dem mindestens einen Wärmeaustauschmantel (106, 306) verbunden ist, und die ferner eine Rückführungsleitung (202, 402) umfasst, die zum Ausströmen des Fluids hydraulisch mit dem mindestens einen Wärmeaustauschmantel (106, 306) verbunden ist, wobei sich der Wärmeaustauschmantel (106, 306) von einer oberen Ebene (203, 403) des Tanks (100, 300) bis zu einer unteren Ebene (204, 404) des Tanks (100, 300) entwickelt und wobei die Zuführungsleitung (201, 401) auf der oberen Ebene (203, 403) mit dem mindestens einen Wärmeaustauschmantel (106, 306) verbunden ist und wobei die Rückführungsleitung (202, 402) auf der unteren Ebene (204, 404) mit dem mindestens einen Wärmeaustauschmantel (106, 406) verbunden ist.

9. Tank nach Anspruch 8, wobei sich die obere Ebene (203, 403) an der seitlichen Wand (101) auf einer Höhe befindet, die in Bezug auf eine Höhe, auf der sich die untere Ebene (204, 404) befindet, größer ist, wenn der Tank (100, 300) in einer Arbeitsstellung aufrecht ist.

10. Tank nach Anspruch 8 oder 9, der ferner ein erstes Ventil (205, 405) mit automatischer Absperrung an der Rückführungsleitung (202, 402) umfasst.

11. Tank nach Anspruch 10, der ferner eine Hauptzuführungsleitung (211, 411) umfasst, die mit der Zuführungsleitung (201, 401) verbunden ist, und die ferner ein zweites Ventil (206, 406) an der Zuführungsleitung (201, 401) umfasst, wobei die Hauptzuführungsleitung (211, 411) und das zweite Ventil (206, 406) dafür konfiguriert sind, ein Ausströmen von Gas kontinuierlich durch das zweite Ventil (206, 406) und die Hauptzuführungsleitung (211, 411) zu ermöglichen.

12. Tank nach einem der Ansprüche 8 bis 11, wobei das Fluid ein Kühlmittelfluid ist, das, auf der oberen Ebene (203, 403), eine niedrigere Temperatur hat und das, auf der unteren Ebene (204, 404) eine höhere Temperatur hat, nachdem es einen Inhalt des Tanks (100, 300), vorzugsweise eine Fermentationsflüssigkeit, gekühlt hat.

13. Tank nach einem der Ansprüche 1 bis 12, wobei das Metallblech eine Metallblechwendel ist, die entlang der Hauptentwicklungsrichtung (X) gewickelt ist.

## Revendications

1. Cuve (100, 300), en particulier cuve de fermentation, ayant un corps sensiblement cylindrique comprenant :
une paroi latérale (101) constituée d'une tôle enroulée selon une spirale tridimensionnelle,
ladite tôle comportant une direction principale de développement (X) et deux bords latéraux (104a, 104b) alignés par rapport à ladite direction principale de développement (X), lesdits deux bords latéraux (104a, 104b) comprenant des premiers segments soudés à des deuxièmes segments desdits deux bords latéraux (104a, 104b) respectivement, lesdits premiers segments et lesdits deuxièmes segments étant adjacents dans ladite spirale tridimensionnelle pour fermer ladite paroi latérale (101),
ladite cuve (100, 300) comprenant en outre au moins une chemise d'échange thermique (106, 306) associée à ladite paroi latérale (101), pour la circulation d'un fluide à l'intérieur de ladite chemise d'échange thermique (106, 306), de manière à échanger de la chaleur à travers ladite paroi latérale (101) avec un contenu de ladite cuve (100, 300).

2. Cuve selon la revendication 1, dans laquelle ladite au moins une chemise d'échange thermique (106, 306) est agencée sur ladite tôle et est alignée par rapport à ladite direction principale de développement (X), étant ainsi enroulée selon ladite spirale tridimensionnelle.

3. Cuve selon la revendication 2, dans laquelle ladite au moins une chemise d'échange thermique (106, 306) comprend une pluralité de spires sur ladite paroi latérale (101), le long de ladite spirale tridimensionnelle, comprenant de préférence au moins une spire complète.

4. Cuve selon l'une quelconque des revendications 1 à 3, dans laquelle ladite chemise d'échange thermique (106, 306) est appliquée sur ladite tôle, notamment en étant soudée.

5. Cuve selon la revendication 4, dans laquelle lesdits deux bords latéraux (104a, 104b) de ladite tôle sont ultérieurement soudés pour constituer ladite paroi latérale (101), ladite tôle comprenant déjà ladite chemise d'échange thermique (106, 306) sur un côté extérieur.

6. Cuve selon l'une quelconque des revendications 1 à 5, dans laquelle lesdits deux bords latéraux (104a, 104b) sont reliés en étant enroulés selon ladite spirale tridimensionnelle et sont soudés, de préférence en continu, de préférence de manière automatisée et de préférence par soudage TIG ou Plasma ou Laser.

7. Cuve selon l'une quelconque des revendications 1 à 6, dans laquelle ladite spirale tridimensionnelle présente un pas d'hélice égal ou supérieur à une distance entre lesdits deux bords latéraux (104a, 104b).

8. Cuve selon l'une quelconque des revendications 1 à 7, comprenant en outre une conduite d'alimentation (201, 401) reliée hydrauliquement à ladite au moins une chemise d'échange thermique (106, 306) pour l'arrivée dudit fluide, et comprenant en outre une conduite de retour (202, 402) connectée hydrauliquement à ladite au moins une chemise d'échange de chaleur (106, 306) pour l'écoulement dudit fluide, ladite chemise d'échange de chaleur (106, 306) se développant à partir d'un niveau supérieur (203, 403) de ladite cuve (100, 300) jusqu'à un niveau inférieur (204, 404) de ladite cuve (100, 300), et ladite conduite d'alimentation (201, 401) étant reliée à ladite au moins une chemise d'échange de chaleur (106, 306) au niveau dudit niveau supérieur (203, 403), et ladite conduite de retour (202, 402) étant connectée à ladite au moins une chemise d'échange de chaleur (106, 406) audit niveau inférieur (204, 404).

9. Cuve selon la revendication 8, dans laquelle ledit niveau supérieur (203, 403) est situé sur ladite paroi latérale (101) à une hauteur qui est supérieure par rapport à une hauteur à laquelle se situe ledit niveau inférieur (204, 404), lorsque ladite cuve (100, 300) est dressée dans une position de travail.

10. Cuve selon la revendication 8 ou la revendication 9, comprenant en outre une première vanne (205, 405) à obturation automatique sur ladite conduite de retour (202, 402).

11. Cuve selon la revendication 10, comprenant en outre une conduite d'alimentation principale (211, 411) reliée à ladite conduite d'alimentation (201, 401), et comprenant en outre une deuxième vanne (206, 406) sur ladite conduite d'alimentation (201, 401), ladite conduite d'alimentation principale (211, 411) et ladite deuxième vanne (206, 406) étant configurées pour permettre l'écoulement de gaz en continu à travers ladite deuxième vanne (206, 406) et ladite conduite d'alimentation principale (211, 411).

12. Cuve selon l'une quelconque des revendications 8 à 11, dans laquelle ledit fluide est un fluide réfrigérant qui, audit niveau supérieur (203, 403), est à une température inférieure et qui, audit niveau inférieur (204, 404), est à une température plus élevée, après avoir réfrigéré un contenu de ladite cuve (100, 300), de préférence un liquide en fermentation.

13. Cuve selon l'une quelconque des revendications 1 à 12, dans laquelle ladite tôle métallique est une bobine de tôle métallique enroulée selon ladite direction principale de développement (X).
